(19) Europäisches Patentamt
European Patent Office
Office européen des brevets

(11) Publication number: 0 252 551 B1

(12) **EUROPEAN PATENT SPECIFICATION**

(45) Date of publication of the patent specification: **30.05.90**

(51) Int. Cl.[5]: **C11D 3/00**, C11D 3/30, A61K 7/08

(21) Application number: **87201203.4**

(22) Date of filing: **24.06.87**

(54) **Softening agents.**

(30) Priority: **05.07.86 GB 8616464**

(43) Date of publication of application: **13.01.88 Bulletin 88/2**

(45) Publication of the grant of the patent: **30.05.90 Bulletin 90/22**

(84) Designated Contracting States: **AT BE CH DE FR GB GR IT LI LU NL SE**

(56) References cited:
**EP-A- 13 450**
**EP-A- 168 889**
**EP-A- 0 023 367**
**EP-A- 0 133 804**
**EP-A- 0 210 704**
**FR-A- 2 389 671**

(73) Proprietor: **THE PROCTER & GAMBLE COMPANY, One Procter & Gamble Plaza, Cincinnati Ohio 45202(US)**
(84) Designated Contracting States: **AT BE CH DE FR GB GR IT LI LU NL SE**

(73) Proprietor: **Procter & Gamble European Technical Center (Naamloze Vennootschap), Temselaan 100, B-1820 Strombeek-Bever(BE)**
(84) Designated Contracting States: **BE DE FR IT LU NL**

(72) Inventor: **Raemdonck, Hans, Belgielaan 12, B-9820 St-Denijs Westrem(BE)**
Inventor: **Busch, Alfred, Luitberg 10, B-1820 Strombeek-Bever(BE)**

(74) Representative: **Suslic, Lydia et al, Procter & Gamble European Technical Center N.V. Temselaan 100, B-1820 Strombeek-Bever(BE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



ACTORUM AG

## Description

### Technical Field

The present invention relates to means of treating fibers and fabrics, wherein a complex of aryl amines and fatty acids or phosphate esters is used to provide softness and anti-static benefits, and wherein the softening agent does not impair the overall cleaning performance on oily or greasy soils. Detergent compositions containing said softening agents are disclosed. The compositions herein can be used to clean and soften fabrics, and to "condition" fibers, especially hair.

### Background

The use of cationic materials to treat hair in a post-shampoo rinse is a well-known cosmetic practice. Likewise, the use of softeners to treat fabrics after a washing operation is a well-known laundering practice. Fabric softeners are, in the main, water-insoluble cationic materials that are incompatible with anionic detersive surfactants used in most fabric washing compositions. For that reason, the softening operation is generally carried out in the laundry rinse bath after the surfactant has been removed from the washing machine. This entails additional work for the user.

Formulators of fabric laundering compositions have long sought means whereby fabric washing and softening could be done concurrently. The same is true for shampoo formulators, since the problems are rather similar. Laundering methods employing clay softeners, mixtures of clays and various amine materials and the like, are described in the following patent applications: German 29/64114.3, 28/57163.3, 24/39541.3, 23/34899.4 and European0 023 367, 0 026 528, 0 028 432. The use of mixtures of amines and soaps (salt of fatty acids) as through-the-wash softeners is disclosed in U.K. Patent 1.514.276.

European Patent Applications 0 133 804 and 0 168 889 relate to complexes of fatty acids and phosphate esters with various fatty amines as fabric softeners and hair conditioners.

The art also describes the use of various cationic materials in sheet form. See U.S. Patent 4.220.562.

The art also teaches the softener used of alkyl phosphonate and quaternary ammonium compounds. See European Patent Application 0 006 268, filed June 11, 1979.

The art also teaches various mixtures of phosphate esters in detergents with fabric softening action - See BE-A 802,679.

The present invention employs novel amine complexes, wherein the amine contains an aryl substituent, in laundry or shampoo compositions to provide cleaning and softening concurrently. The softening agents herein provide softening and anti-static benefits, especially when used with clay softeners. Importantly, the use of the aryl amines enhances softening over the usual alkyl amines. Contrary to the usual alkyl amines, the softening agents herein do not have any negative interaction with surface-active agents, and the overally cleaning performance on greasy stains is thus maintained to a very good level.

### Summary of the Invention

The present invention encompasses detergent or shampoo compositions comprising detersive surfactants, optional conventional ingredients, and a softening agent, characterized in that the softening agent is constituted of a water-insoluble, water-dispersible aryl amine complex of the type disclosed in more detail, hereinafter.

Typical detergent or shampoo compositions in accordance with this invention are those wherein the level of said complex is at least 0.1% (preferably 1% to 15%) by weight of the total composition.

The laundry detergent compositions herein may contain, in addition, a clay fabric softener, as well as various detergency builders such as phosphate, nitrilotriacetate, polycarboxylate, citrate and zeolite builders, or mixtures thereof, as well as other typical adjuvant ingredients.

Liquid, solid and granular compositions are contemplated herein, as well as compositions which are releasably combined with non-particulate substrates.

The invention also includes the novel aryl amine complexes of the type described more fully hereinafter.

The ingredients and means for preparing the compositions are disclosed more fully hereinafter. All weights and proportions are by weight of composition, unless otherwise specified.

### Detailed Description of the Invention

As noted hereinafter, the composition of this invention comprise, in major part, conventional ingredients that are quite familiar to formulators of laundry and shampoo compositions. One of the major advantages of the softening agents used herein is that they are entirely compatible with such conventional laundry ingredients, used at conventional concentrations.

Fatty Acid-Aryl Amine Softening Agent

These materials are one type of water-insoluble, water-dispersible softening agent used herein, and are characterized in that they consist of a complex of

a) an amine of the formula

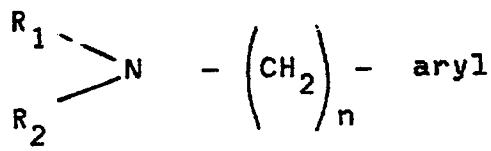

wherein $R_1$ and $R_2$ are $C_{1-22}$ alkyl or alkenyl groups, and the sum of carbon atoms in $R_1 + R_2$ is at least 14, and n is an integer of 1 to 5, preferably 1, with
b) a fatty acid of the formula RCOOH with R being a $C_{8-20}$ alkyl group.

Phosphate Ester Aryl Amine Softening Agent

These materials are the second type of water-insoluble, water-dispersible softening agent used herein, and are characterized in that they consist of a complex of

a) an amine of the formula

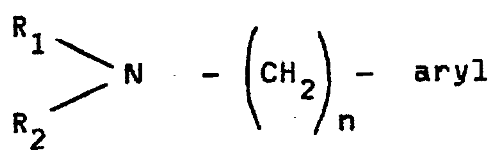

wherein $R_1$ and $R_2$ are $C_{1-22}$ alkyl or alkenyl groups, and the sum of carbon atoms in $R_1 + R_2$ is at least 14, and n is an integer of 1 to 5, preferably 1, with
b) a phosphate ester of the formula

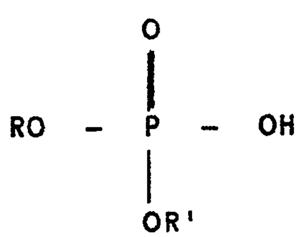

or

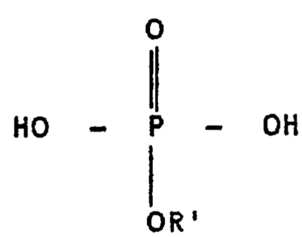

wherein R and R′ are $C_1$-$C_{20}$ alkyl or ethoxylated alkyl groups of the general formulae: alkyl -$(OCH_2CH_2)_y$, wherein the alkyl substituent is $C_1$-$C_{20}$, and y is an integer of 1 to 15.

Amines

The amines employed herein are of the formula

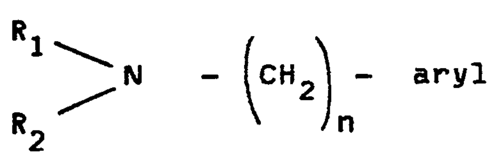

wherein $R_1$ and $R_2$ are selected independently $C_{1-22}$ alkyl or alkenyl groups, the sum of carbon atoms in $R_1 + R_2$ being at least 14, and n being an integer of 1 to 5, n is preferably 1, and the aryl group is preferably phenyl. In a preferred execution, $R_1$ and $R_2$ each contain from 12 to 20 carbon atoms.

Most preferred amines for use herein are di($C_{12-20}$) benzylamines, like di-tallow benzylamine or dicoconut benzylamine.

Fatty Acids

The fatty acids used herein are of the formula RCOOH, with R being a $C_8$ to $C_{20}$, preferably $C_{12}$ to $C_{20}$, alkyl group.

Examples of fatty acids include nonanoic, lauric, myristic, palmitic, stearic, oleic, and mixtures thereof.

Phosphate Esters

The phosphate esters used herein are commercially available materials of the general formulae:

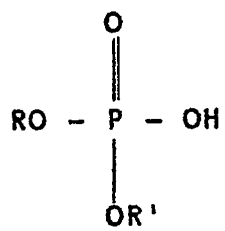

and

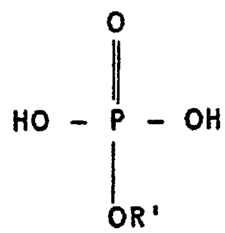

wherein R and R′ are $C_1$–$C_{20}$ alkyl or (preferably) ethoxylated alkyl groups of the general formulae : alkyl-$(OCH_2CH_2)_Y$, wherein the alkyl substituent is $C_1$–$C_{20}$, preferably $C_8$–$C_{16}$ and y is an integer of 1 to 15, preferably 2–10, most preferably 2–5. Such compounds are prepared by known methods from phosphorus pentoxide, phosphoric acid or phosphorus oxy halide and alcohols or ethoxylated alcohols.

It will be appreciated that the formulae depicted represent mono- and di-esters, and commercial phosphate esters will generally comprise mixtures of the mono- and di-esters, together with some proportion of tri-ester. Typical commercial esters are available under the Trade Marks "Phospholan"® PDB3 (Diamond Shamrock) and "Servoxyl"® VPAZ (Servo).

Preparation of Fatty Acid-Aryl Amine Softening Compositions

The aryl amine-fatty acid complexes are prepared separately from the balance of the composition, and are preferably then added to the conventional detergent ingredients in such a way as to ensure that the complexes are homogeneously dispersed therein as microfine particles. This can most conveniently be done by preparing a melt of the fatty acid and the aryl amine, maintaining the melt stage for about ten minutes whereby the complex forms, dispersing the molten complex into a stirred, aqueous crutcher mix comprising the balance of the detersive ingredients, and spray-drying in standard fashion. In alternate mode, the melt can be atomized onto the detergent granules or allowed to solidify, ground in a colloid mill, and dry-mixed with the balance of the detergent composition.

The weight ratio of aryl amines to fatty acids is generally superior to 1:1, preferably from 3:1 to 10:1.

The aryl amine fatty acid complexes are characterized by their microfine particle size, i.e., preferred microfine particles substantially all pass through a Millipore (TM) filter of 10 micrometers size, and the majority of particles pass through a 7 micrometers filter.

The complexes are further characterized by their melting points, which lie generally in the range of 32–65°C.

While not intending to be limited by theory, it appears that, in-use, the heat of the wash water (30–90°C) softens the particle and disrupts hydrogen bonding. The particle deposits on fabrics, to provide a lubricious feel.

Preparation of Phosphate Ester-Aryl Amine Softening Composition

In general terms, the aryl amine-phosphate ester softening agents are prepared separately from the balance of the compositions, and are preferably then added to the other, conventional ingredients to provide the final formulations. This can most conveniently be done by preparing a melt of the phosphate ester and the amine and maintaining the melt stage for a few minutes. The melt can then be layered onto a sheet substrate to prepare, for example, a dryer-added fabric softener. To prepare a granular detergent, the melt is preferably sprayed onto the spray-dried granules comprising the balance of the detersive ingredients. The melt can also be sprayed on a particulate carrier, such as perborate monohydrate, and then added as particles or prills to the rest of the ingredients.

For the preferred aryl amine- phosphate ester softening agents herein, the weight ratio of aryl amine:phosphate ester is above 5:1, preferably 7:1 to 15:1, most preferably 10:1.

An especially preferred softening agent herein is prepared by melting together ten parts by weight of di-$C_{12}$–$C_{18}$ benzyl amine and one part by weight of a mono - $C_{12}$–$C_{14}$ $(EO)_{1-5}$ ester of phosphoric acid, sold under the Trade Mark "Servoxyl® VPAZ".

Usage levels of the aryl amine softening agent

The aryl amine softening agents herein will generally be used at levels of at least 0.1%, preferably 1% to 15%, in detergent compositions; at levels of at least 1%, preferably 1% to 25% in rinse-added fabric softeners; and at levels of 2% to 90% in dryer- and wash machine- added sheets (where the balance of the composition mainly comprises the weight of the sheet substrate).

Softener Clay

The above-disclosed softening agents are most preferably used in granular detergent compositions, where they are most preferably used in combination with a detergent-compatible clay fabric softener. Such clay softeners are well-known in the detergency patent literature and are in broad commercial use, both in Europe and in the United States. Included among such clay softeners are various heat-treated kaolins and various multi-layer smectites. Preferred clay softeners are smectite softener clays that are described in German patent document 23 34 899 and in U.K. Patent 1.400.898 which can be referred to for details. Softener clays are used in the preferred compositions at levels of at least 1%, generally 1-20%, preferably 2-7%.

Detersive Surfactants

-The detergent compositions of this invention will contain organic surface-active agents ("surfactants") to provide the usual cleaning benefits associated with the use of such materials.

Detersive surfactant useful herein include well-known synthetic anionic, amphoteric and zwitterionic surfactants. Typical of these are the alkyl benzene sulfonates, alkyl- and alkylether sulfates, paraffin sulfonates, olefin sulfonates, amine oxides, -sulfonates of fatty acids and of fatty acid esters, and the like, which are well-known from the detergency art. In general, such detersive surfactants contain an alkyl group in the $C_9$-$C_{18}$ range; the anionic detersive surfactants can be used in the form of their sodium, potassium or triethanolammonium salts. McCUTCHEON's INDEX contains detailed listings of such typical detersive surfactants. $C_{11}$-$C_{16}$ alkyl benzene sulfonates, $C_{12}$-$C_{18}$ paraffin-sulfonates and alkyl sulfates are especially preferred in the compositions of the present type.

Also useful herein as surfactants are the water-soluble soaps, e.g. the common sodium and potassium coconut or tallow soaps well-known in the art.

It is to be understood that the use of typical alkoxylated nonionic surfactants (e.g. the $C_9$-$C_{18}$ alkyl alcohols and alkyl phenols with 5 to 20 ethoxyl groups) is preferably limited in the practice of this invention to levels of not more than about 5%, preferably not more than 2%, most preferably 0-1%, of the compositions when clay is present as a co-softener, since alkoxylates can interfere with the softening properties of clay. In clay-free compositions, the alkoxylated nonionics can be used at any desired level.

The surfactant component can comprise as little as 1% of the laundry detergent compositions herein, but generally the compositions will contain 5% to 40%, preferably 6% to 30%, of surfactant. Mixtures of the anionics, such as the alkyl benzene sulfonates, alkyl sulfates and paraffin sulfonates are preferred for through-the-wash cleansing of a broad spectrum of soils and stains from fabric.

Detersive Adjuncts

The compositions herein can contain other ingredients which aid in their cleaning performance. For example, it is highly preferred that through-the-wash detergent compositions contain a detergent builder and/or metal ion sequestrant. Compounds classifiable and well-known in the art as detergent builders include the nitrilotriacetates, polycarboxylates, citrates, water-soluble phosphates such as tri-polyphosphate and sodium ortho- and pyro-phosphates, silicates, and mixtures thereof. Metal ion sequestrants include all of the above, plus materials like ethylene diaminetetraacetate, the amino-polyphosphonates and phosphates (DEQUEST)® and a wide variety of other poly-functional organic acids and salts too numerous to mention in detail here. See U.S. Patent 3 579 454 for typical examples of the use of such materials in various cleaning compositions. In general, the builder/sequestrant will comprise 0.5% to 45% of the composition. The 1–10 micrometer size zeolite (e.g. zeolite A) builders disclosed in German Patent 2 422 655 are especially preferred for use in low-phosphate compositions which contain the softeners described herein.

The laundry compositions herein also preferably contain enzymes to enhance their through-the-wash cleaning performance on a variety of soils and stains. Amylase and protease enzymes suitable for use in detergents are well-known in the art and in commercially available liquid and granular detergents. Commercial detersive enzymes (preferably a mixture of amylase and protease) are typically used at levels of 0.001% to 2%, and higher, in the present compositions.

Other highly desirable detergent ingredients for use in the detergent compositions of the present invention are quaternary ammonium compounds of the form $R_4R_5R_6R_7N^+X^-$, wherein $R_4$ is alkyl having from 10 to 20, preferably from 12-18 carbon atoms, and $R_5$, $R_6$ and $R_7$ are each $C_1$ to $C_4$ alkyl preferably

methyl: $X^-$ is an anion, e.g., chloride. Examples of such quaternary ammonium compounds include $C_{12}$-$C_{14}$ alkyl trimethyl ammonium chloride and cocoalkyl trimethyl ammonium methosulfate. The quaternary ammonium compounds can be used at levels from 0.5% to 5%, preferably from 1% to 3%.

Moreover, the compositions herein can contain, in addition to ingredients already mentioned, various other optional ingredients typically used in commercial products to provide aesthetic or additional product performance benefits. Typical ingredients include pH regulants, perfumes, dyes, bleaches, optical brighteners, soil suspending agents, hydrotropes and gel-control agents, freeze-thaw stabilizers, bactericides, preservatives, suds control agents, bleach activators and the like.

Form of the compositions

In a through-the-wash laundry mode, the compositions are typically granular, and used at a concentration of at least 500 ppm, preferably 0.10% to 2.5%, in an aqueous laundry bath at pH 7-11 to launder fabrics. The laundering can be carried out over the range from 5°C to the boil, with excellent results.

In an alternate mode, the softening agents herein may be releasably adsorbed or releasably coated onto a non-particulate substrate such as a non-woven or paper sheet or flexible sponge mat, or the like. Such sheet-form objects may be added to the laundry or rinse bath, or to the laundry dryer, where the softener is released to provide fabric softening. In an alternate, and highly preferred, mode the softening agents are used in sheet form in combination with a bleach activator (such as tetraacetyl ethylene diamine or a straight- or branched-chain $C_6$-$C_{10}$ oxybenzene sulfonate) as a combined perborate-activator and softener in a laundry liquor. See, for example U.S. Patent 4.220.562.

Such sheet-form products will generally employ 1-20 grams of the softening agent herein 1-20 grams of the bleach activator.

In still another mode, the softening agents herein can be formulated as a liquid detergent, or as a liquid fabric softener and used in a post-laundry rinse bath. Such liquid softeners can comprise, for example, a simple dispersion of the softening agent in water or water-alcohol.

Preferred compositions

Highly preferred laundry detergents with through-the wash fabric softening benefits are those wherein the amine is ditallowbenzylamine, the complex is present at a level of from 1 to 5% by weight, the composition contains from 2% to 7% by weight of a smectite clay softener, and the composition is in the form of spray-dried granules.

Industrial Application

The following examples are typical of the compositions of this invention but are not intended to limit the scope of the invention.

Example I

A mix of stearic acid (total 1.5% of complete formulation) and di-hardened tallow benzyl amine (total 5% of complete formulation) are admixed, melted in a jacketed batch, and maintained as a melt for about 10 minutes (excess heating may cause yellowing).

A standard aqueous crutcher mix comprising the following ingredients is prepared (percentages listed relate to percent ingredients in the complete formulation after spray-drying).

| Ingredients | Percent |
|---|---|
| $C_{11-12}$ alkyl benzene sulfonate | 6.2 |
| Tallow alcohol ethoxylate (EO11) | 1.0 |
| Sodium perborate | 20.0 |
| Sodium tripolyphosphate | 24.0 |
| Sodium sulfate | 19.0 |
| Sodium silicate | 8.0 |
| Smectite clay* | 2.4 |
| Carboxymethyl cellulose | 0.4 |
| Polyacrylate (soil suspender) | 1.7 |
| Enzymes | 0.5 |
| Optical brightener | 0.23 |
| Sulphonated zinc phthalocyanine** | 25 ppm |
| EDTA | 0.2 |
| Perfume/copper salts/minors | 0.5 |
| Moisture | to 100 |

* Natural smectite; ion exchange capacity above 50 meq/100 g clay
** U.S. Patent 3 927 967

The pre-formed complex is poured into the stirred crutcher mix (60-90°C) as the final ingredient. (In general it is preferred to add the complex to the crutcher after most of the ingredients have been added and thoroughly blended.) The crutcher mix-plus-complex is then handled in entirely standard fashion, and spray-dried to form the final composition.

The composition of Example I is free-flowing and exhibits excellent through-the-wash fabric softening performance when fabrics washed therewith are line-dried.

Moreover, the composition of Example I exhibits good cleaning performance on greasy stains, when compared to a composition containing usual alkyl amine softening agents.

Performance data

The composition of Example I was compared for through-the-wash softeness vs. an identical composition containing a ditallow methyl amine/stearic acid premix instead of the premix of the invention (reference I) and with an identical composition wherein the arylamine/stearic acid complex of the invention was replaced by additional sodium sulfate). (Reference II) The design of the test was such as to compare softness of textile pieces laundered 4 times (multi-cycle) with invention and reference composition.

The testing conditions were as follows:

- automatic drum washing machine MIELE 423
- heating from 15°C to 60°C; 50 minutes at 60°C
- 1% product concentration in wash liquor
- 15 grains/gallon (0.31g/l) water hardness (3:1 Ca/Mg ratio).

The washed and line dried swatches were compared by a panel of two expert judges, working independently, by a paired comparison technique using a 9-point Scheffe scale. Differences were recorded in panel score units (psu), positive being performancewise better.

The testing results were as follows:

| Softness: Invention vs. Reference I | |
|---|---|
| Cotton fabric | +1.0* |
| Polyester fabric | +1.2* |
| Softness: Invention vs. Reference II | |
| Cotton fabric | +1.7* |
| Polyester fabric | +1.3* |

* statistically significant

The composition of Example I was also compared for cleaning benefits with Reference I. Results were als follows:

| | psu |
|---|---|
| greasy stain removal | + 1.2* |

Similar results can be obtained when ditallowbenzylamine is replaced by dicoconutbenzyl amine.

EXAMPLE II

Ditallow benzyl amine (DTBA) (total 5% of complete formulation after spray-drying) and monococonutalkyl(ethoxy)1-5 phosphoric acid ester (0.5% of complete formulation) are admixed, melted in a jacketed bath, and maintained as a melt for about ten minutes. Stirring assures homogeneity.

A standard aqueous crutcher mix comprising the following ingredients is prepared (percentages listed relate to percent ingredients in the complete formulation after spray-drying).

| Ingredients | Percent |
|---|---|
| $C_{11-12}$ alkyl benzene sulfonate | 6.2 |
| Tallow alcohol ethoxylate (EO11) | 1.0 |
| Sodium perborate | 20.0 |
| Sodium tripolyphosphate | 24.0 |
| Sodium sulfate | 20.5 |
| Sodium silicate | 8.0 |
| Smectite Clay* | 2.4 |
| Carboxymethyl cellulose | 0.4 |
| Polyacrylate (soil suspender) | 1.7 |
| Enzymes | 0.5 |
| Optical brightener | 0.23 |
| Sulphonated zinc phthalocyanine** | 25 ppm |
| EDTA | 0.2 |
| Perfume/copper salts/minors | 0.5 |
| $C_{12-24}$ alkyl trimethylammonium chloride | 1.9 |
| Moisture | to 100 |

* Natural smectite; ion exchange capacity above 50 meq/100 g clay
** U.S. Patent 3 927 967

The crutcher mix is handled in entirely standard fashion, and spray-dried. The pre-formed softener is then sprayed onto the spray-dried granules.

The composition of Example II is free-flowing and provides excellent through-the-wash fabric softening when used at laundry concentration of 0.1% and above, and better cleaning on greasy stains when compared with similar composition containing usual alkyl-amine based softening agent.

In an alternate mode, the amine-phosphate ester softener can be added thereto as particles or "prills"

EXAMPLES III–IV

The following compositions are also prepared:

| Ingredients | Ex. III Percent | Ex. IV |
|---|---|---|
| Zeolite A (1–10 micrometer) | 26.0 | 26.0 |
| Sodium nitrilotriacetate | 5.0 | 5.0 |
| Smectite clay* | 3.0 | 3.0 |
| DTBA.fatty acid complex** | 6.3 | – |
| DTBA.phosphate ester complex*** | – | 5.2 |
| $C_{11-12}$ alkyl benzene sulfonate (Na) | 6.5 | 6.5 |
| Tallow ethoxylate (EO 9–11) | 1.0 | 0.5 |
| Sodium perborate $4H_2O$ | 20.0 | 20.0 |
| Sodium silicate | 8.0 | 8.0 |
| CMC | 1.0 | 1.0 |
| Sodium sulfate | 20.0 | 20.0 |
| Enzymes (1:1 amylase/protease) | 1.5 | 1.5 |
| Optical brightener | 0.5 | 0.5 |
| TAED | 1.2 | 1.2 |
| Water, minors | to 100 | to 100 |

DTBA = ditallow benzyl amine
* As Gelwhite GP (TM); $CaCO_3$ ion exchange capacity 70 meq/100 g.
** Prepared as in Ex. I.
*** Prepared as in Ex. II.

The above compositions are prepared by spray-drying the aqueous crutcher mix. It is preferred that TAED and enzymes be dry-mixed with the balance of the composition, after spray-drying. In use, the compositions give excellent through-the-wash fabric softening performance, without impairing the overall cleaning performance on greasy stains.

In an optional mode, the above compositions may be modified by removing the clay and replacing it with an equivalent amount of DTBA. fatty acid complex or DTBA-phosphate ester complex.

EXAMPLE V

A laundry additive product is prepared by warming 6.5g of DTBA and 0.8g of tetradecylphosphate to form a melt, and spreading the melt onto an ordinary disposable paper hand-towel (20x20 cm). 4 grams of TAED powder (1–10 micrometer) are sprinkled onto, and pressed into, the melt before it has the chance to solidify.

The article of Example V is added to a laundry liquor containing a commercial perborate/clay detergent composition (DASH-3; Trade Mark) to enhance through-the-wash softening.

EXAMPLE VI

The article of Example V is modified by deleting the TAED and replacing the Example V softener with 3.5g of the aryl amine-phosphate ester softening agent of Example II. The resulting article is tumbled with damp fabrics in a standard hot air clothes dryer, whereby the softener is transferred to the fabrics to impart softness.

EXAMPLE VII

A fine-fabric laundering composition with fabric softening properties is as follows :

| Ingredients | Percent |
|---|---|
| $C_{10-14}$ Alkyl Sulfate | 9.0 |
| Tallow alkyl benzene sulfonate | 2.0 |
| Coconut Soap | 4.0 |
| Glycerine | 3.0 |
| DTBA/Phosphate ester complex* | 6.0 |
| Triethanolamine | to pH 7.0 |
| Perfume | 0.25 |
| Water | to 100 |
| * DTBA (15 parts)/bis-decylphosphate (1 part) as melt. | |

The composition of Example VII is in the form of a "milky" liquid.

## Claims

1. A detergent or shampoo composition comprising detersive surfactants, optional conventional ingredients, and a softening agent, characterized in that the softening agent is constituted by a water-insoluble, water-dispersible complex of:
a) an amine of the formula

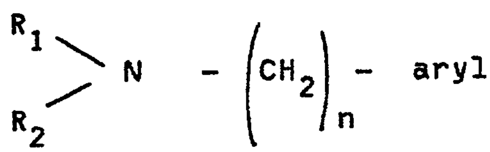

wherein $R_1$ and $R_2$ are $C_{1-22}$ alkyl or alkenyl group, the sum of carbon atoms in $R_1 + R_2$ is at least 14, and n is an integer of 1 to 5 with
b) a compound selected from
I) fatty acids of the formula RCOOH with R being a $C_{8-20}$ alkyl group.
II) phosphate esters of the formulae

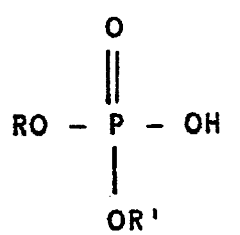

and

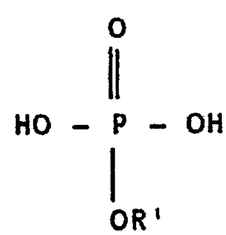

wherein R and R′ are $C_1$-$C_{20}$ alkyl or ethoxylated alkyl groups of the general formulae: alkyl -$(OCH_2CH_2)_y$, wherein the alkyl substituent is $C_1$-$C_{20}$, and y is an integer of 1 to 15.

2. A composition in accordance with claim 1 wherein the level of said complex is at least 0.1% by weight of the total composition.

3. A composition in accordance with claim 1 and 2 wherein the level of said complex is from 1% to 15% by weight of the total composition.

4. A composition in accordance with claim 1 wherein the aryl group in said amine is phenyl.

5. A composition in accordance with claim 1 wherein said amine is a di-($C_{12}$-$C_{20}$ alkyl)benzylamine.

6. A composition in accordance with any of the foregoing claims which contains in addition a clay softener.

7. A composition according to any of the foregoing claims which contains a detergent builder selected from phosphate, nitrilotriacetate, polycarboxylate, citrate and zeolite builders, or mixtures thereof.

8. A composition in accordance with any of the preceding claims wherein the amine is di-$C_{12}$-$C_{20}$ benzylamine, the complex is present at a level of from 1 to 5% by weight, the composition contains from 2% to7% by weight of a smectite clay softener, and the composition is in the form of spray-dried granules.

9. A composition in accordance with claim 1-7, which is in liquid form.

10. A composition in accordance with claim 1-7 which is releasably combined with a non-particulate substrate.

11. A water insoluble, water-dispersible softening agent, characterized in that it consists of a complex of

a) an amine of the formula

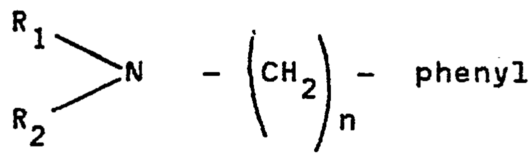

wherein $R_1$ and $R_2$ are $C_{1-22}$ alkyl or alkenyl group, and the sum of carbon atoms in $R_1 + R_2$ is at least 14, and n is an integer of 1 to 5 with

b) a fatty acid of the formula RCOOH with R being a C8 to 20 alkyl group.

12. A water-insoluble, water-dispersible softening agent, characterized in that it consists of a complex of

a) an amine of the formula

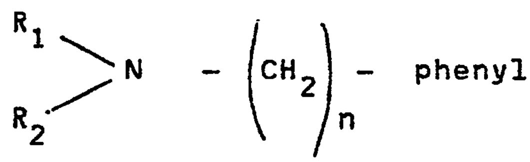

wherein $R_1$ and $R_2$ are $C_{1-22}$ alkyl or alkenyl group, and the sum of carbon atoms in $R_1 + R_2$ is at least 14, and n is an integer of 1 to 5 with

b) a phosphate ester of the formula

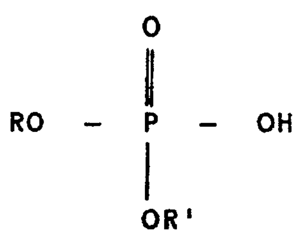

or

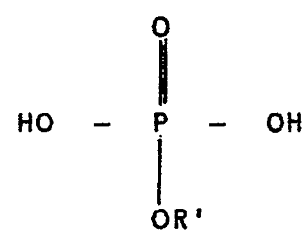

wherein R and R′ are $C_1$–$C_{20}$ alkyl or ethoxylated alkyl groups of the general formulae: alkyl -$(OCH_2CH_2)_y$, wherein the alkyl substituent is $C_1$–$C_{20}$, and y is an integer of 1 to 15.

## Patentansprüche

1. Detergens- oder Shampoozusammensetzung, umfassend detersive grenzflächenaktive Mittel, fakultative herkömmliche Bestandteile und einen Weichmacher, dadurch gekennzeichnet, daß der Weichmacher durch einen wasserunlöslichen, wasserdispergierbaren Komplex von:

a) einem Amin der Formel

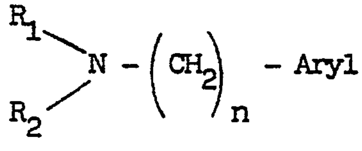 ,

worin $R_1$ und $R_2$ $C_{1-22}$-Alkyl- oder -Alkylengruppen sind, die Summe der Kohlenstoffatome in R1 + $R_2$ mindestens 14 beträgt und n eine ganze Zahl von 1 bis 5 ist, mit

b) einer Verbindung, ausgewählt unter

I) Fettsäuren der Formel RCOOH, worin R eine $C_{8-20}$-Alkylgruppe ist,

II) Phosphatestern der Formeln

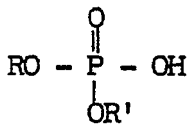

und

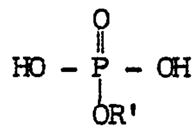

,

worin R und R′ $C_1$–$C_{20}$-Alkyl- oder ethoxylierte Alkylgruppen der allgemeinen Formeln: Alkyl-$(OCH_2CH_2)_y$ darstellen, worin der Alkylsubstituent $C_1$–$C_{20}$ ist, und y eine ganze Zahl von 1 bis 15 bedeutet,

gebildet wird.

2. Zusammensetzung nach Anspruch 1, worin die Menge an dem genannten Komplex mindestens 0,1 Gew.-% der Gesamtzusammensetzung beträgt.

3. Zusammensetzung nach Anspruch 1 und 2, worin die Menge an dem genannten Komplex von 1 Gew.-% bis 15 Gew.-% der Gesamtzusammensetzung beträgt.

4. Zusammensetzung nach Anspruch 1, worin die Arylgruppe im genannten Amin Phenyl ist.

5. Zusammensetzung nach Anspruch 1, worin das genannte Amin ein Di-($C_{12}$–$C_{20}$-alkyl)benzylamin ist.

6. Zusammensetzung nach einem der vorherstehenden Ansprüche, welche zusätzlich einen Tonweichmacher enthält.

7. Zusammensetzung nach einem der vorherstehenden Ansprüche, welche einen Detergensgerüststoff, ausgewählt unter Phosphat-, Nitrilotriacetat-, Polycarboxylat-, Citrat- und Zeolithgerüststoffen, oder Gemische hievon, enthält.

8. Zusammensetzung nach einem der vorherstehenden Ansprüche, worin das Amin Di-$C_{12}$–$C_{20}$-benzylamin ist, der Komplex in einer Menge von 1 bis 5 Gew.-% vorliegt, die Zusammensetzung von 2 Gew.-% bis 7 Gew.-% eines Smectittonweichmachers enthält und die Zusammensetzung in der Form von sprühgetrockneten Körnchen vorliegt.

9. Zusammensetzung nach den Ansprüchen 1 bis 7, welche in flüssiger Form vorliegt.

10. Zusammensetzung nach den Ansprüchen 1 bis 7, welche mit einem nicht-teilchenfömigen Substrat freisetzbar verbunden ist.

11. Wasserunlöslicher, wasserdispergierbarer Weichmacher, dadurch gekennzeichnet, daß er aus einem Komplex von

a) einem Amin der Formel

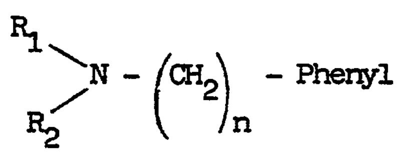

,

worin R1 und R2 $C_{1-22}$-Alkyl- oder -Alkenylgruppen sind und die Summe der Kohlenstoffatome in $R_1$ + $R_2$ mindestens 14 beträgt, und n eine ganze Zahl von 1 bis 5 bedeutet, mit

b) einer Fettsäure der Formel RCOOH, worin R eine $C_{8-20}$-Alkylgruppe ist,

besteht.

12. Wasserunlöslicher, wasserdispergierbarer Weichmacher, dadurch gekennzeichnert, daß er aus einem Komplex von

a) einem Amin der Formel

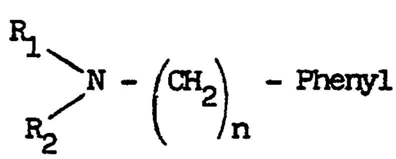

,

worin $R_1$ und $R_2$ $C_{1-22}$-Alkyl- oder -Alkenylgruppen sind und die Summe der Kohlenstoffatome R1 + R2 mindestens 14 beträgt, und n eine ganze Zahl von 1 bis 5 bedeutet, mit

b) einem Phosphatester der Formel

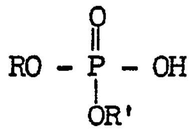 oder 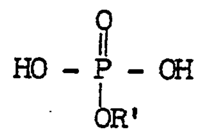 ,

worin R und R′ $C_1$–$C_{20}$-Alkyl oder ethoxylierte Alkylgruppen der allgemeinen Formeln Alkyl-($OCH_2CH_2$)y darstellen, worin der Alkylsubstituent $C_1$-$C_{20}$ ist, und y eine ganze Zahl von 1 bis 15 bedeutet,

besteht.

**Revendications**

1. Composition détergente ou de shampooing comprenant des agents tensio-actifs détergents, des ingrédients classiques facultatifs, et un agent adoucissant, caractérisée en ce que l'agent adoucissant est constitué par un complexe insoluble dans l'eau dispersable dans l'eau, de

a) une amine de formule

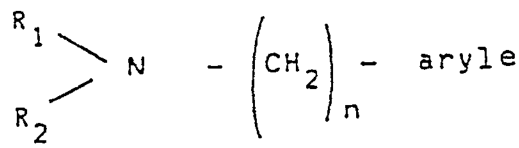

où $R_1$ et $R_2$ représentent des groupes alkyle ou alkényle en $C_1$ à $C_{22}$, la somme des atomes de carbone dans $R_1$ + $R_2$ est d'au moins 14, et n est un nombre entier allant de 1 à 5 avec

b) un composé choisi parmi

I) les acides gras de formule RCOOH où R est un groupe alkyle en $C_8$ à $C_{20}$,

II) les esters de phosphate de formules

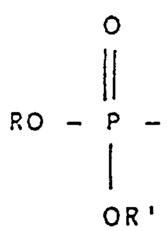 et 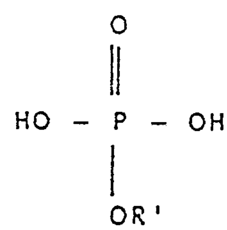

où R et R′ représentent des groupes alkyle en $C_1$ à $C_{20}$ ou des groupes alkyle éthoxylés de formules générales: alkyl-($OCH_2CH_2$)y,

où le substituant alkyle est en $C_1$ à $C_{20}$, et y est un nombre entier allant de 1 à 15.

2. Composition selon la revendication 1 où le niveau dudit complexe est au moins d'environ 0,1% en poids de la composition totale.

3. Composition selon les revendications 1 et 2 où le niveau dudit complexe est de 1% à 15% en poids de la composition totale.

4. Composition selon la revendication 1 dans laquelle le groupe aryle dans ladite amine est le phényle.

5. Composition selon la revendication 1 dans laquelle ladite amine est une di-(alkyl en $C_{12}$ à $C_{20}$)-benzylamine.

6. Composition selon l'une quelconque des revendications précédentes qui contient en outre un adoucissant à base d'argile.

7. Composition selon l'une quelconque des revendications précédentes qui contient un auxiliaire de détergence choisi parmi les auxiliaires de détergence à base de phosphate, nitrilotriacétate, polycarboxylate, citrate et zéolithe, ou leurs mélanges.

8. Composition selon l'une quelconque des revendications précédentes dans laquelle l'amine est la dibenzylamine en $C_{12}$ à $C_{20}$, le complexe est présent à un niveau allant de 1 à 5% en poids, la composition contient de 2% à 7% en poids d'un adoucissant à base d'argile smectite, et la composition est sous la forme de granules séchés par pulvérisation.

9. Composition selon les revendications 1–7, qui est sous forme liquide.

10. Composition selon les revendications 1–7 qui est combinée de façon libérale avec un substrat non particulaire.

11. Agent adoucissant insoluble dans l'eau, dispersable dans l'eau, caractérisé en ce qu'il consiste en un complexe de

a) une amine de formule

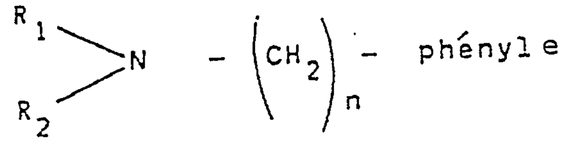

où $R_1$ et $R_2$ représentent un groupe alkyle ou alkényle en $C_1$ à $C_{22}$, et la somme des atomes de carbone dans $R_1 + R_2$ est d'au moins 14, et n est un nombre entier allant de 1 à 5, avec

b) un acide gras de formule RCOOH où R est un groupe alkyle en $C_8$ à $C_{20}$.

12. Agent adoucissant insoluble dans l'eau, dispersable dans l'eau, caractérisé en ce qu'il consiste en un complexe de

a) une amine de formule

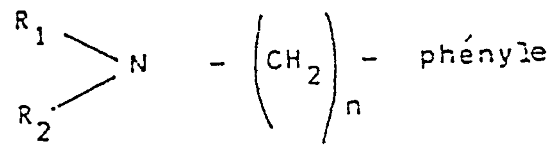

où $R_1$ et $R_2$ représentent un groupe alkyle ou alkényle en $C_1$ à $C_{22}$, et la somme des atomes de carbone dans $R_1 + R_2$ est d'au moins 14, et n est un nombre entier allant de 1 à 5, avec

b) un ester de phosphate de formule

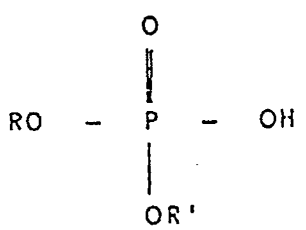

ou

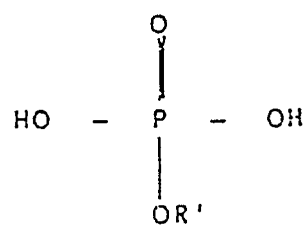

où R et R' représentent des groupes alkyle en $C_1$ à $C_{20}$ ou des groupes alkyle éthoxylés de formules générales: alkyl-$(OCH_2CH_2)_y$, où le substituant alkyle est en $C_1$ à $C_{20}$, et y est un nombre entier allant de 1 à 15.